# EUROPEAN PATENT APPLICATION

(11) **EP 2 241 321 A1**
(43) Date of publication of application: **20.10.2010**
(21) Application number: 09707915.6
(22) Date of filing: 09.02.2009
(51) Int. Cl.: A61K 33/14, A61P 31/12, A61L 2/16

(54) **NOVEL HYDROXY RADICAL GENERATION METHOD, AND ANTI-VIRAL MATERIAL UTILIZING HYDROXY RADICAL GENERATED BY THE METHOD**

(30) Priority: 08.02.2008 WO PCT/JP2008/052601
(71) Applicant: Mochigase Co., Ltd., Tottori 689-1201 (JP); National University Corporation Tokyo Medical and Dental University, Bunkyo-ku Tokyo 113-8510 (JP)
(72) Inventor: YAMAMOTO, Norio, Chiba-shi Chiba 262-0015 (JP); WAKABAYASHI, Kazuo, Yazu-gun Tottori 689-1402 (JP)
(74) Representative: HOFFMANN EITLE
(86) International application number: PCT/JP2009/000516
(87) International publication number: WO 2009/098908

(57) **Abstract**

Disclosed is a novel hydroxy radical generation method which relies on a novel scientific fact relating to the generation of a hydroxy radical and the inactivation of a virus. Also disclosed is an anti-viral material produced by utilizing the method. The anti-viral material comprises a metal oxide powder and a hydroxide which enable the generation of a hydroxy radical that can inactivate a virus. An virus can be inactivated by the action of the hydroxy radical generated from the anti-viral material.

## Description

### TECHNICAL FIELD

The present invention relates to a method for efficiently generating hydroxyl radicals and to an anti-viral material that thoroughly and definitively inactivates viruses with the utilization of the hydroxyl radicals generated by the method.

### RELATED ART

A hydroxyl radical, which is represented as · OH, is a radical derived from a hydroxy group, and is one of the molecular species called active oxygen. Among active oxygen, hydroxyl radicals have high reactivity and strong oxidizing power, and thus the radicals are known to react with protein, lipid, glucide, nucleic acid (DNA, RNA), and the like, and especially known to sequentially oxidize lipid.

Such properties of the hydroxyl radicals have been used to conduct many studies on methods for purifying noxious organic materials contained in the air and water. These methods for generating hydroxyl radicals generally include the Fenton reaction (reaction in which hydrogen peroxide and divalent iron ions are reacted under acidic conditions to produce hydroxyl radicals), the Haber-Weiss reaction (reaction in which hydrogen peroxide and superoxide anions are reacted in the presence of trivalent iron ions to produce hydroxyl radicals), the method in which hydrogen peroxide is irradiated with ultraviolet rays, the method in which water is irradiated with ozone and ultraviolet rays, the method in which corona or plasma discharge is generated in water-rich gas or in water (JP-A-2001-70946, JP-A-2000-288547), and similar methods.

However, the above-mentioned methods for generating hydroxyl radicals are required to generate hydroxyl radicals under conditions that can be dangerous to the human body, including the use of hydrogen peroxide, irradiation of ultraviolet rays and ozone, use of corona or plasma discharge, or the like. Therefore, it has been desired to develop a method for more safely and readily generating hydroxyl radicals.

Other means for generating hydroxyl radicals include a method that utilizes a silver-carrying photocatalyst (for example, JP-A-2004-337562). It is known that the hydroxyl radicals generated by such a method inactivate viruses and bacteria.

However, when the silver-carrying photocatalyst is used to inactivate viruses, the photocatalyst is required to be irradiated with light of a certain level of intensity such as natural or fluorescent light. Therefore, it has been desired to develop a method that can generate hydroxyl radicals with or without light irradiation to inactivate viruses and bacteria. Also, improvements can still be made in terms of costs, disposal convenience, increased efficiency of hydroxyl radical generation or control of the generated amount of hydroxyl radicals, and the like.

For anti-viral materials using other known methods for inactivating viruses, their mechanism of inactivating of viruses is unclear and their probability of inactivation is low. They also have many limitations on the means for applying the anti-viral agents to their target viruses (hereinafter referred to as application means). The following anti-viral agents (a) and (b) used by an ion and a gas method, respectively, are the agents referred to as having a relatively clear inactivation mechanism, and are also similar in that the effect of inactivation of viruses is unclear and that there are limitations on the details and the types of the application means.

(a) There is disclosed an anti-viral agent of a two component system of cationic groups such as quaternary ammonium base having anti-viral activity and hydrocarbon chains (for example, saturated fatty acid) (see Japanese Patent No. 3222471).

The probability of inactivation is low because, in its mechanism of inactivation of viruses, the hydrocarbon chains attract hydrophobic viral envelopes, and the cationic groups near the viruses inactivate the enveloped viruses (paramyxoviruses, coronaviruses, poxviruses, etc.).

Moreover, for the products applied with anti-viral properties, the anti-viral agents are covalently bound and immobilized to fabric, from which protective products, articles worn or used by health care workers (wound and burn coverings), patient care articles (sutures and dressings), and the like are made. Therefore, there are limitations on the application means.

(b) There is disclosed an anti-viral agent that inactivates molds, bacteria, and viruses by chlorine dioxide gas, which is widely used as bleach, disinfectant, and the like (see Japanese Patent No. 3547140).

The mechanism of inactivation of viruses in this case, however, is a mechanism in which:
1) a hydrophilic material blended with a source of chlorite anions (chlorite salts, etc.) is included in hydrophobic particles, allowing moisture adsorbed by the hydrophobic particles to be introduced to the inside of the hydrophobic particles, and
2) the chlorite salts or the like are hydrolyzed by the introduced moisture to release hydronium ions, and the hydronium ions react with the chlorite anions in the hydrophilic particles to release chlorine oxide gas to inactivate viruses.
Therefore, the probability of inactivation of viruses is unknown and the application means are limited.
As described above, the related-art anti-viral agents have a mechanism of inactivation of which details are unknown with unclear probability of inactivation of viruses and limited application means even when they are used by the ion method, the gas method, or another method.

The term "application means" as used in the following description of the invention is used interchangeably with the application means of the related art of anti-viral agents.

WO2005/013695 A1 describes that when dolomite is calcined, water is poured to the calcined dolomite while the material is still at a high temperature to partially hydrate the dolomite. The resultant dolomite is pulverized or sieved into powder with a particle size in the range from 0.1 µm to 60 µm inclusive (these powder particles are the secondary particles, which are agglomerates of the primary particles, while the primary particles constituting the secondary particles, have a size in the range from 1 nm to 200 nm inclusive), and the resultant powder has anti-viral action. However, no examination has been made in terms of the generation of hydroxyl radicals, and thus the details of the mechanism of inactivation of viruses and the probability of inactivation of viruses are still unknown.

In view of the foregoing, the present inventors have closely examined, by experiment, a method for more safely, readily, and efficiently generating hydroxyl radicals, the mechanism of activation of viruses, and the concrete means for inactivating viruses. As a result, the inventors have found several scientific facts about the inactivation of viruses and have obtained the invention.

### DISCLOSURE OF THE INVENTION

An object of the invention is to provide a method for more safely, readily, and sufficiently generating hydroxyl radicals than the known methods for generating hydroxyl radicals, such as the Fenton reaction.

Another object of the invention is to provide, based on the fact that hydroxyl radicals efficiently inactivate viruses, an anti-viral material that contains metal oxide powder and a hydroxide that allow the generation of hydroxyl radicals.

In order to fulfill the objects described above, the invention provides the following.
(1) A method for generating hydroxyl radicals including contacting powder of an oxide of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide.
(2) The method according to (1), in which both the powder of the metal oxide and the hydroxides are contained in slaked dolomite obtained by calcining dolomite and partially hydrating the calcined dolomite.
(3) The method according to (2), in which the slaked dolomite is obtained by calcining a dolomite raw material at a temperature from 700°C to 1300°C for 1 to 20 hours, cooling the calcined material to room temperature, and then contacting the cooled material with 35 to 60 parts by weight of water based on 100 parts by weight of the dolomite.
(4) The method according to (3), in which the calcination of the slaked dolomite is performed by heating the dolomite to a temperature from 700°C to 1000°C, at a temperature increase rate of 5 to 10°C/min and maintaining the temperature for 8 to 12 hours, while air is circulated intermittently.
(5) The method according to (1) or (2), in which the weight ratio of the powder of the metal oxide to the hydroxides, i.e., (powder of the metal oxide)/(hydroxides) is in the range from 0.001 to 100.
(6) The method according to (1), in which the powder of the metal oxide is powder of one or more oxides selected from magnesium oxide, calcium oxide, manganese dioxide, iron (II) oxide, iron (III) oxide, copper oxide, zinc oxide, or aluminum oxide, and the hydroxides are one or more hydroxides selected from sodium hydroxide, potassium hydroxide, magnesium hydroxide, calcium hydroxide, barium hydroxide, aluminum hydroxide, or ammonium hydroxide.

The invention also provides the following:
(7) An anti-viral material produced by having a method in which hydroxyl radicals are generated by contacting powder of an oxide of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide.

The method for generating hydroxyl radicals in the invention can safely, readily, and efficiently generate hydroxyl radicals without employing conditions that can be dangerous to the human body, such as hydrogen peroxide, ultraviolet rays, and corona discharge.

An anti-viral material produced by having the method for generating hydroxyl radicals in the invention can be used as an anti-viral material for the prevention of viral infection in a variety of applications, such as masks, drapes, and protective clothing.

### DESCRIPTION OF THE PREFERRED EMBODIMENTS

### SUMMARY OF THE INVENTION

The invention is based on the novel fact that hydroxyl radicals are generated by contacting powder of oxides of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide.

In addition, an anti-viral material with anti-viral properties that is produced by having the method in which hydroxyl radicals are generated by contacting oxides of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide in the invention is based on the novel scientific facts that have been newly found by the inventors regarding the inactivation of viruses by hydroxyl radials, such as the following facts (1) to (5).
(1) The fact that hydroxyl radicals are highly effective in independently inactivating viruses.
(2) The fact that active oxygen other than hydroxyl radicals are not or not very effective in independently inactivating viruses.
(3) The fact that hydroxyl radicals can inactivate various viruses against which the radical's mechanism of inactivation of viruses works effectively
(4) The fact that the combination of metal oxide powder with a hydroxide and control of the reaction allow the effect of hydroxyl radicals in the inactivation of viruses to be improved.
(5) The fact that the surface condition of the metal oxide powder affects the generation of hydroxyl radicals in inactivating viruses.

The invention will be described in detail.

### <Metal oxide>

The powder of oxides of one or more metals selected from the group consisting of alkali metals, alkaline earth metals, metals of groups 4 to 12 of the periodic table, or aluminum according to the invention can generate hydroxyl radicals when it reacts with a hydroxide. The oxides may be natural (typically, a metal oxide contained in a mineral) or synthetic, and may be used individually, or in combinations of two or more. With regard to the powder of the metal oxides, it is preferred to use metal oxides as the raw materials that are converted into powder with a larger specific surface area, especially porous powder, in terms of efficiency for the generation of hydroxyl radicals.

The powder of the natural metal oxides includes one produced by chemical treatment, physical treatment, or the like of a mineral (for example, a mineral containing a salt or a double salt). The metal oxides derived from a mineral must be made into the metal oxide powder that can generate hydroxyl radicals when it reacts with a hydroxide (see the Examples described below).

Among the metal oxides described above, for example, magnesium oxide, calcium oxide, copper oxide, zinc oxide, iron oxide, silver oxide, aluminum oxide, and the like are preferred in terms of the reactivity with a hydroxide. In particular, when the metal oxides include magnesium oxide or calcium oxide as a basic metal oxide, it is easy to generate hydroxyl radicals and to inactivate viruses by virtue of the generated hydroxyl radicals.

Even when the metal oxides are contained in a mineral, pulverization, chemical treatment, physical treatment, powderization, and the like of the mineral allow the metal oxides to be a metal oxide powder that originates from the mineral or mineral powder that contains the metal oxides (hereinafter may be referred to as metal oxide powder originating from a mineral or the like), which are available for the reaction.

However, the metal oxide powder originating from a mineral or the like must be able to generate hydroxyl radicals when it reacts with a hydroxide. The mineral powder containing the metal oxides may also contain another mineral component as long as it does not inhibit the reaction for the generation of hydroxyl radicals.
Examples of the mineral available for use include dolomite minerals, tourmaline minerals (for example, dravite, schorl, and elbaite), zeolite minerals, kaolin minerals, quartz porphyry, and other minerals. Pulverization, chemical treatment, physical treatment, powderization, and the like depending on the mineral allow the mineral to be made into a metal oxide powder, a combination of metal oxide powder and hydroxide powder, or a combination of metal oxide powder, hydroxide powder, and a third component powder.

### <Hydroxide>

The hydroxides used in the invention can be used individually, or in combinations of two or more, as long as hydroxide ions can be provided when hydroxyl radicals are generated by the reaction with the metal oxide powder. Examples of such hydroxides include one or more hydroxides selected from alkali metal hydroxides, alkaline earth metal hydroxides, iron hydroxide, copper hydroxide, zinc hydroxide, aluminum hydroxide, or ammonium hydroxide. In terms of smooth and easy generation of hydroxyl radicals, sodium hydroxide, potassium hydroxide, magnesium hydroxide, calcium hydroxide, and the like are preferred.

Examples of the use of the hydroxides include the use of sodium hydroxide, potassium hydroxide, magnesium hydroxide, or calcium hydroxide, and an aqueous solution thereof, while examples of the use of the hydroxides in combination include the use of a mixture of sodium hydroxide and potassium hydroxide and an aqueous solution thereof, and a mixture of magnesium hydroxide and calcium hydroxide, and an aqueous solution thereof.

The hydroxides can be used in the form of a solution (for example, aqueous sodium hydroxide), slurry (for example, slurry containing sodium hydroxide), a solid (for example, anhydrous sodium hydroxide), and other forms.

Even when the hydroxides are in the form of a solid, a reaction site such as a water layer is formed by adsorption of water onto the metal oxide powder and deliquescence caused by the reaction of the solid hydroxide (for example, anhydrous sodium hydroxide), and therefore, a reaction generating hydroxyl radicals is engendered.

When the hydroxides are aqueous solutions, a concentration of, for example, 0.001 to 0.8 mol/l (preferably 0.005 to 0.5 mol/l) is employed to provide alkalinity for the reaction, with the alkalinity facilitating the reaction for the generation of hydroxyl radicals.

An alkaline earth metal hydroxide alone can generate hydroxyl radicals without contact with a metal oxide. Such an alkaline earth metal hydroxide can be used in the form of a solution, slurry, a solid or the like, and when it is used in the form of a solid, adsorption of water and the like drive a reaction for the generation of hydroxyl radicals. The diameter of the primary particles of the alkaline earth metal hydroxide is preferably in the range from 1 nm to 1000 nm inclusive, more preferably from 1 nm to 400 nm inclusive, and particularly preferably from 1 nm to 200 nm inclusive. Among such alkaline earth metal hydroxides, magnesium hydroxide and calcium hydroxide are preferred in terms of the generated amount of hydroxyl radicals and the efficiency for the generation of hydroxyl radicals.

### <Reaction for generation of hydroxyl radicals>

Examples of the specific method for generating hydroxyl radicals by contacting metal oxide powder with a hydroxide include a method in which metal oxide powder is mixed in an aqueous solution or slurry of a hydroxide to react the mixture, a method in which metal oxide powder and a hydroxide are added to a protic or non-protic organic solvent to react the mixture, and a method in which metal oxide powder and a solid hydroxide are brought into contact to react them at a reaction site formed by the adsorbed water.

The reaction for the generation of hydroxyl radicals proceeds when the surface of the metal oxide powder is surrounded by the alkaline atmosphere of the hydroxide, and the change in the intensity of the alkaline atmosphere caused by adjusting the alkaline concentration allows the amount and the rate of the generation of hydroxyl radicals to be controlled.

The amount ratio of the metal oxide powder to the hydroxide when they are brought into contact is preferably in the range from 0.001 to 100, and more preferably in the range from 0.01 to 10, when it is measured as the weight ratio of (powder of the metal oxide)/(hydroxide). By adjusting the ratio within the range as defined above, it is possible to efficiently generate hydroxyl radicals.

For example, when the metal oxide is magnesium oxide and the hydroxide is magnesium hydroxide, the weight ratio of (powder of the metal oxide)/(hydroxide) is preferably 0.1 to 9. When the metal oxide is magnesium oxide and the hydroxide is calcium hydroxide, the weight ratio of (powder of the metal oxide)/ (hydroxide) is preferably 0.1 to 4.

When the metal oxide is magnesium oxide and the hydroxide is a mixture of magnesium hydroxide and calcium hydroxide, the weight ratio of (powder of the metal oxide)/(hydroxide) is preferably in the range from 0.1 to 2.5.

In the method for generating hydroxyl radicals in the invention, an additive can also be added in addition to the metal oxide powder and the hydroxide in order to more efficiently promote and control the generation of hydroxyl radicals. For example, titanium oxide, SrTO₃, Ag-NbO₂, AgGaO₂, or the like can be used as an additive, when needed.

When hydroxyl radicals are generated using the method according to the invention, the metal oxide powder and the hydroxide may be prepared separately to react them as described above, or a mineral that contains both a metal oxide and hydroxide may be used without modification.

Examples of the use of a mineral that contains both a metal oxide and hydroxide include the use of the slaked powder obtained by the processes of calcination and hydration (slaking) of a dolomite mineral (a double salt of calcium carbonate and magnesium carbonate (Ca · Mg(CO₃)₂)). Due to the fact that the processes of calcination and hydration of a dolomite mineral are performed under a special operational condition to produce a mixture of the metal oxide and the hydroxide that cause a reaction for the generation of hydroxyl radicals, it is possible to make the mixture into powder for use.

When a dolomite mineral is calcined, dolomite raw materials are heated to a temperature from 700°C to 1300°C inclusive and preferably from 700°C to 1000°C inclusive under atmospheric pressure and at a temperature increase rate ranging from 1°C/min to 15°C/min inclusive and preferably from 5°C/min to 10°C/min inclusive, followed by retaining the above-defined temperature range for from 1 hour to 20 hours inclusive, and preferably from 8 hours to 12 hours inclusive. During calcination, CO₂ gas generated by thermal decomposition of the dolomite influences the decomposition behavior. When the concentration of the CO₂ gas is high, the decomposition reaction occurs at a high temperature. Conversely, when the concentration of the CO₂ gas is low, the decomposition reaction occurs at a low temperature. In order to facilitate the decomposition reaction, it is necessary to adjust the airflow, and thus the air is preferably circulated intermittently.

Next, after cooling the dolomite to which the calcination process has been applied to room temperature (20°C ±15°C (JIS Z 8703)), the dolomite is brought into contact with 35 to 60 parts by weight of water, preferably 45 to 50 parts by weight of water based on 100 parts by weight of the dolomite to partially hydrate (slake) the calcined dolomite. The time period for the contact with water is preferably between 5 hours and 20 hours, and the amount of water in the slaked dolomite (slaked powder) after the slaking process is preferably in the range from 1 to 5% by weight.
The slaked powder described above contains magnesium oxide (MgO), calcium hydroxide (Ca(OH)₂), and magnesium hydroxide (Mg(OH)₁₋₂) as reactive constituents, and the constituents that may be used are desirably calcium carbonate and trace constituents. When other constituents are produced during the calcination and hydration processes, the reaction resulting in the generation of hydroxyl radicals may be inhibited. Moreover, when the amount of the magnesium oxide decreases, the generated amount of hydroxyl radicals also decreases. In the slaked powder, the content of MgO is 2 to 22% by weight and preferably 5 to 15% by weight, the content of Ca(OH)₂ is 40 to 60% by weight and preferably 45 to 55% by weight, and the content of Mg(OH)₁₋₂ is 5 to 25% by weight and preferably 10 to 20% by weight.

In order to more efficiently generate hydroxyl radicals, the slaked dolomite described above has preferably the diameter of the secondary particles in the range from 0.1 µm to 60 µm, more preferably in the range of 0.1 µm to 10 µm, and particularly preferably in the range from 0.1 µm to 1 µm. In particular, when the diameter of the secondary particles is 1 µm or less, hydroxyl radicals are more prominently generated. Accordingly, the inventors have confirmed that this dramatically improves the anti-viral effect.

In the metal oxide powder (especially, powder of oxides of alkali earth metal elements) and the powder containing the metal oxides that originate from the mineral containing both the metal oxide and the hydroxide in the invention, 60% or more of the unit volume of the metal oxide powder has a specific surface area of 20 m²/g or more, and more preferably 40 m²/g or more as measured by the BET method. The case of a powder with a large specific surface area (m²/g) of 80 m²/g or more that is difficult to be made into a powder is more readily engendered and thus facilitates the reaction for the generation of hydroxy radicals. Even when the specific surface area is less than 20m²/g, the reaction may occur, but it is difficult to generate hydroxyl radicals.

The "unit area" of the powder means a given unit area determined by the sampling of the pulverized powder, rather than an artificial mixture of powder with different particle diameters. The "quantitatively principal component of the metal oxide powder" in the invention is a component that constitutes a significant fraction of the unit volume of the metal oxide powder, the fraction being, for example, 60% or more of the unit volume.

### <Identification of Hydroxyl Radicals>

The hydroxyl radicals were examined and identified by the following methods including quantitation.
(a) A method in which hydroxyl radicals are reacted with an active oxygen detection reagent that uses 2-[6-(4-amino)phenoxy-3H-xanthen-3-on-9-yl]benzoic acid (APF) or 2-[6-(4-hydroxy)phenoxy-3H-xanthen-3-on-9-yl]benzoic acid (HPF) to determine the quantity from the fluorescence intensity of the produced compound with strong fluorescence (fluorescein)
(b) A method in which ethanol and hydroxyl radicals are reacted to produce hydroxyethyl radicals, and the produced hydroxyethyl radicals are trapped by POBN (α-(4-pyridyl-1-oxide)-N-tertbutylnirone) to detect the radicals by Electron Spin Resonance (ESR)
(c) A method in which the generation and presence of hydroxyl radicals are recognized by determining whether the purple color of the radical scavenger 1,1-diphenyl-2-picrylhydrazyl (DPPH) is decolorized when hydroxyl radicals are identified

### <Applying method for generating hydroxyl radicals according to the Invention to anti-viral material>

The term "anti-viral material" in the invention means fibers and plastics to which an anti-viral effect is imparted by using the hydroxyl radicals generated by the method for generating hydroxyl radicals according to the invention, and various products such as masks and protective clothing, and other various applied products such as medicines that are made of such fibers and plastics. When an anti-viral effect is imparted to such a wide variety of applied products (for example, when the effect is imparted by a method such as attachment, adhesion, fixation, support, and mixing), it is desirable that there are little or no limitations in imparting the anti-viral effect. In this regard, the invention allows an anti-viral effect to be imparted to a wide variety of applied means by using solid powder as the generation source of hydroxyl radicals, and thus it becomes possible to use a broad range of anti-viral materials with almost no limitations.

In the case where magnesium oxide powder as the metal oxide powder is brought into contact with aqueous sodium hydroxide as the hydroxide, the generation of a large quantity of hydroxyl radicals has been confirmed by the determination method that uses the APF active oxygen detection reagent, and the detection method in which ethanol and hydroxyl radicals are reacted to produce hydroxyethyl radicals, whereby the produced hydroxyethyl radicals are selectively trapped by POBN to detect the radicals by ESR.

The inventors have made several assumptions about the reaction mechanism of the generation of hydroxyl radicals including, for example, a single-step reaction mechanism, a two-step reaction mechanism, and a reaction mechanism in which hydrogen peroxide is produced in an intermediate stage.

### <Mechanism of inactivation of viruses>

The invention has revealed a phenomenon in which hydroxyl radicals destroy the structure of viruses, a phenomenon in which hydroxyl radicals cause aggregation of virus proteins, a phenomenon in which hydroxyl radicals cause polymerization of virus proteins, and a phenomenon in which hydroxyl radicals cause change in the proteins projecting from the surface of a virus, resulting in the formation of large chunks or populations, by which the viruses are inactivated (see the Examples described below).

### <Target Viruses>

Any of the viruses can be inactivated by the anti-viral material according to the invention, as long as hydroxyl radicals destroy the structure of the viruses, hydroxyl radicals cause an agglomeration phenomenon of the proteins projecting from the surface of the viruses, and hydroxyl radicals cause an aggregation phenomenon of the virus proteins.

Examples of a part of the target viruses include influenza viruses (for example, highly-pathogenic avian influenza viruses (H5N1 HPAIV)/Vietnam strains and Hong Kong strains), coronaviruses (for example, SARS virus), flaviviruses (for example, hepatitis C virus, dengue virus, Japanese B encephalitis virus, West Nile virus, and yellow fever virus), picornaviruses (for example, poliovirus and hepatitis A virus), caliciviruses (for example, norovirus), filoviruses (for example, Ebola virus and Marburg virus), rhabdoviruses (for example, rabies virus), paramyxoviruses (for example, measles virus and mumps virus), herpesviruses, papillomaviruses, polyomaviruses, adenoviruses, parvoviruses, retroviruses (for example, human immunodeficiency virus), and hepadnaviruses (for example, hepatitis B virus).

### <Application Means>

The means for applying the anti-viral material allow an anti-viral property to be imparted to a region where a human or the animal might come into contact with viruses, resulting in inactivation of the viruses. When using the powder that is effective for inactivating various viruses and that is easy to use as anti-viral material, the application means have no particular limitations on the usage, form, size, method of use, and other characteristics.

The application means are used for, for example, diagnostic instruments, extracorporeal circulation devices, protective articles, clinical test instruments (for example, gloves, various test instruments, sterile cloths, masks, instrument covers, and bandages), hospital articles (for example, surgical gowns, protective cloths, sterile cloths, masks, instrument covers, and bandages), medical supplies (for example, bandages and masks), articles for home medical care (for example, bedclothes and others), hygienic materials, articles for medical and health care, hospital buildings, food manufacturing plants, containers, and food packaging materials, in a manner that the function of inactivating viruses can be effective.

The application means may be, for example, a pharmaceutical carrier (in the form of solid, liquid, paste, or the like), a pharmaceutical composition, or another application means for pharmaceutical preparations. Solid carriers include china clay (kaolin), sucrose, crystalline cellulose, talc, agar and the like.

### <Embodiments of application means>

The anti-viral material is provided to application means in a manner that hydroxyl radicals can be generated. For example, the material is provided by methods including attachment, adhesion, application, fixation, inclusion, support, and others. When the hydroxide is in a solution form, the anti-viral material may be included in the application means. In addition, the hydroxide is prepared separately, and then the previously prepared hydroxide is reacted with the metal oxide that is provided to the application means to generate hydroxyl radicals. In this case, the anti-viral material of the invention includes the metal oxide that is provided to the application means and the previously prepared hydroxide.

Furthermore, when viruses exist in an environment containing the hydroxide, it is not necessary to previously prepare the hydroxide, and it is possible to react the hydroxide in the environment containing viruses with the metal oxide that is provided to the application means to generate hydroxyl radicals that can inactivate viruses.

In the invention, modifications or partial changes, and additions may be optionally made, as long as they are suitable for the objects of the invention and do not significantly inhibit the advantages of the invention. In addition, such modifications, changes, and additions are within the scope of the invention. For example, the invention can be applied as a pharmaceutical agent (an antibacterial agent, for example) against other organisms, in which the agent is able to cause destruction, aggregation, or the like of the organisms by virtue of the generated hydroxyl radicals in accordance with the principles of the invention.

The invention will be described in detail based on the Examples. The Examples are part of the specific embodiments, and thus they are not intended to limit the scope of the invention.

### EXAMPLES

### Example 1 Verification of hydroxyl radicals

Magnesium oxide (MgO) powder was charged in a 0.1 mol/l sodium hydroxide (NaOH) aqueous solution, and the mixture was reacted. Subsequently, the resultant mixture was reacted with an aminophenyl fluorescein (APF) reagent (reagent for detection of active oxygen), and thus, the presence of hydroxyl radicals (· OH) was confirmed and the generation of a large amount of hydroxyl radicals (· OH) was confirmed by quantification using a calibration curve.

### Example 2 Verification of hydroxyl radicals

Magnesium oxide (MgO) powder was charged in a 0.1 mol/l sodium hydroxide (NaOH) aqueous solution, and ethanol and POBN were further added to the mixture. Hydroxyl radicals reacted with ethanol to generate hydroxyethyl radicals, and POBN trapped the hydroxyethyl radicals. An experiment for measuring the trapped hydroxyethyl radicals was performed by electron spin resonance (ESR). The ESR detected a typical peak pattern indicating the generation of hydroxyl radicals.

### Example 3 Verification of reaction between reaction sources coexisting in mineral powder

The generation of hydroxyl radicals (· OH) from mineral powder in which metal oxides and hydroxides coexisted was verified.

A sample obtained by calcining a dolomite mineral containing a double salt of calcium carbonate and magnesium carbonate (Ca · Mg · (CO₃)₂) and by slaking the dolomite mineral was used as a sample of mineral powder in which metal oxides and hydroxides coexisted. Totally different slaked materials are produced from the dolomite mineral depending on the process conditions (a temperature increase rate and the airflow conditions (such as the presence or absence of airflow and the airflow rate)).

Therefore, the experiment was performed under operation conditions that were estimated from the generation mechanism of hydroxyl radicals (· OH) for generating a slaked material containing calcium carbonate (CaCO₃), calcium hydroxide (Ca(OH)₃)₂, magnesium hydroxide (Mg(OH)₁₋₂), and magnesium oxide (MgO) by slaking the calcination. The operation conditions were, for example, as follows: a raw material dolomite was calcined at a temperature from 700°C to 1000°C at a temperature increase rate of 5 to 10°C/min for 10 hours and was cooled down to room temperature, and then was brought into contact with 45 to 50% by weight of water based on the weight of the dolomite. The slaked material was adjusted to become a powder having a specific surface area of 40 m²/g or more as measured by a BET method. Hydroxyl radicals (· OH) were generated from this sample and were able to inactivate viruses.

Although the specific surface area of an anti-viral agent (slaked dolomite) disclosed in WO 2005/013695 A1 was 18.43 m²/g, the specific surface area of the sample described above in the present specification was 40 m²/g or more.

### Example 4 Verification of inactivation of viruses

A verification experiment for the inactivation capability of hydroxyl radicals to SARS viruses (SARS-CoV) was performed by using the plaque reduction method.

The sample of Example 3 containing calcium carbonate (CaCO₃), calcium hydroxide (Ca(OH)₂), magnesium hydroxide (Mg(OH)_{1∼2}), and magnesium oxide (MgO) was used for generating the hydroxyl radicals.

Although at first the control had an infectivity titer of 2,000,000 plaque/mL, the infectivity titer was reduced to zero after hydroxyl radical treatment.

### Example 5 Verification of inactivation of viruses

A verification experiment for the inactivation capability of hydroxyl radicals in relation to SARS viruses was performed using hydroxyl radicals generated from magnesium oxide (MgO) powder and a sodium hydroxide (NaOH) aqueous solution.

Even though at first the control had an infectivity titer larger than that of Example 4 at first, the infectivity titer was reduced to zero after hydroxyl radical treatment.

### Example 6 Verification of inactivation of viruses

Mice in two groups each of which consisted of five mice were prepared. The five mice in one group were allowed to inhale the highly pathogenic avian influenza viruses (H5N1 HPATV)Vietnam strain through their noses. The mice in the other group were allowed to inhale the highly pathogenic avian influenza viruses (H5N1 HPAIV)/Vietnam strain treated with the hydroxyl radicals of Example 3.

Three days after the infection, the mice in the group subjected to no hydroxyl radical treatment had a viral load of 1000 plaque/mL in the nasal lavage fluid. On the other hand, the mice in the group subjected to the hydroxyl radical treatment had a viral load of zero.

Among the mice in the group subjected to no hydroxyl radical treatment, the first one died after 10 days, the next two died after 11 days, the next one died after 12 days, and then the last one died after 13 days.

By contrast, all of the mice in the group subjected to the hydroxyl radical treatment survived even after 14 days.

### Example 7 Verification of inactivation of viruses

A verification experiment for inactivation capability of hydroxyl radicals to highly pathogenic avian influenza viruses (H5N1 HPAIV)/Vietnam strain was performed under the same condition as that of Example 4.
Although at first the control had an infectivity titer of 10,000,000 plaque/mL, the infectivity titer was reduced to zero after hydroxyl radical treatment.

### Example 8 Verification of inactivation of viruses

A verification experiment for the inactivation capability of hydroxyl radicals in relation to highly pathogenic avian influenza viruses (H5N1 HPAIV)/Hong Kong strain was performed under the same conditions as that of Example 4.

Although at first the control had an infectivity titer of 5,000,000 plaque/mL, the infectivity titer was reduced to zero after hydroxyl radical treatment.

### Example 9 Verification of inactivation mechanism of viruses

Anti-IgG antibodies in which anti spike antibodies and gold colloids were bonded and made to interact with projection (spike) proteins existing on the surfaces of SARS virus (SARS-CoV) particles, and the spike proteins on the surfaces of the virus particles were observed through an electron microscope. As a result, the gold colloids were distributed around the virus particles, and the distribution of the gold colloids was the same as that of the spike proteins of the viruses. Subsequently, after the SARS viruses (SARS-CoV) were brought into contact with hydroxyl radicals, Anti-IgG antibodies in which anti spike antibodies and gold colloids were bonded were made to interact in a similar manner, and the viruses were observed through an electron microscope. As a result, the gold colloids were distributed in a place where the proteins were aggregated, grouped, or polymerized, and the breakdown of the viral structure and the inactivation of viruses arising in association with the change of the spike proteins on the surfaces of the virus particles were observed.

### Example 10 Verification of inactivation mechanism of viruses

Western blot was performed using anti spike antibodies on SARS viruses subjected to no hydroxyl radical treatment and on SARS viruses subjected to hydroxyl radical treatment. Electrophoresis was performed without a reducing agent added, and the disappearance of bands corresponding to spike proteins was confirmed on the sample subjected to hydroxyl radical treatment. When a reducing agent was added to perform electrophoresis, then recovery of the bands corresponding to spike proteins was confirmed. This indicates that the spike proteins were oxidized by hydroxyl radicals and thus had a higher-molecular weight.

### Example 11 Verification of inactivation mechanism of viruses

Western blot was performed using anti spike antibodies on SARS viruses subjected to hydroxyl radical treatment and on SARS viruses subjected to hydroxyl radical treatment in a state where a hydroxyl radical remover was added to the generation source of hydroxyl radicals. Electrophoresis was performed without a reducing agent added, and the disappearance of bands corresponding to spike proteins was confirmed on the sample subjected to hydroxyl radical treatment. On the other hand, the recovery of bands corresponding to spike proteins was confirmed on a sample to which a hydroxyl radical remover (sodium salicylate in this case) was added. This indicates that the hydroxyl radical remover inhibited the spike proteins from having higher-molecular weight due to hydroxyl radicals.

### Example 12 Verification of inactivation mechanism of viruses

Conditions for calcining and slaking the dolomite mineral in the preparation of samples in Example 3 were changed to prepare powder of a slaked material containing no magnesium oxide (MgO). In other words, in this Example, raw material dolomite was calcined at a temperature of 700°C or less, cooled down to room temperature, and then was brought into contact with 45 to 50 parts by weight of water based on 100 parts by weight of the dolomite to produce slaked dolomite. This sample of the slaked dolomite containing no magnesium oxide (MgO) failed to inactivate viruses.

### Example 13 Comparison of amount of generated hydroxyl radicals

The viral agent disclosed in WO 2005/013695 A1 was provided to the present inventors by the applicant company of the anti-viral agent disclosed in the same publication, that is, Mochigase Co., Ltd.

Calcium hydroxide (Ca(OH)₂) (purity of 99.90%, manufactured by Wako Pure Chemical Industries, Ltd.), calcium carbonate (CaCO₃) (purity of 99.90%, manufactured by Wako Pure Chemical Industries, Ltd.), magnesium hydroxide (Mg(OH)₂) (purity of 99.90%, manufactured by Wako Pure Chemical Industries, Ltd.), and magnesium oxide (MgO) (purity of 99.90%, manufactured by Wako Pure Chemical Industries, Ltd.) were prepared as reagents for components constituting the viral agent disclosed in WO 2005/013695 A1. Subsequently, a specimen (specimen No. 1) including a phosphoric acid buffer (final concentration of 0.1 M), an HPF reagent (final concentration of 5 µM), and calcium hydroxide (Ca(OH)₂) (standard reagent, final concentration of 50 mM) was prepared.

A specimen (specimen No. 2) including a phosphoric acid buffer (final concentration of 0.1 M), an HPF reagent (final concentration of 5 µM), and calcium carbonate (CaCO₃) (manufactured by Wako Pure Chemical Industries, Ltd., final concentration of 50 mM) was prepared.

A specimen (specimen No. 3) including a phosphoric acid buffer (final concentration of 0.1 M), an HPF reagent (final concentration of 5 µM), and magnesium hydroxide (Mg(OH)₂) (manufactured by Wako Pure Chemical Industries, Ltd., final concentration of 50 mM) was prepared.

A specimen (specimen No. 4) including a phosphoric acid buffer (final concentration of 0.1 M), an HPF reagent (final concentration of 5 µM), and magnesium oxide (MgO) (manufactured by Wako Pure Chemical Industries, Ltd., final concentration of 50 mM) was prepared.

A specimen (specimen No. 5) including a phosphoric acid buffer (final concentration of 0.1 M), an HPF reagent (final concentration of 5 µM), and the viral agent disclosed in WO 2005/013695 A1 provided by Mochigase Co., Ltd. (final concentration of 0.75%) was prepared.

A specimen (specimen No. 6) including a phosphoric acid buffer (final concentration of 0.1 M) and an HPF reagent (final concentration of 5 µM) was prepared as a control.

Subsequently, each of the specimens Nos. 1 to 6 as described above was incubated at room temperature for 15 minutes, and then the fluorescence intensity was measured by a fluorescent plate reader (ARVO MX, manufactured by PerkinElmer Co., Ltd.) to quantify hydroxyl radical production.

The result is given in Table 1.

In Table 1, the viral agent disclosed in WO 2005/013695 A1 provided by Mochigase Co., Ltd. was represented as BRP³ (registered trademark).

**Table 1**

| Specimen | Component | Amount of generated radicals |
|---|---|---|
| No. 1 | Ca(OH)₂ (50 mM) | 6236 |
| No. 2 | CaCO₃ (50 mM) | Less than 10 |
| No. 3 | Mg(OH)₂ (50 mM) | 11907 |
| No. 4 | MgO (50 mM) | 3918 |
| No. 5 | BRP³ (registered trademark) (1%) | 15582 |
| No. 6 | H₂O | Less than 10 |

From Table 1, it was revealed that specimen No. 3 had a large amount of generated radicals after specimen No. 5.

### Example 14 Comparison of amount of generated hydroxyl radicals under shaded condition

A solution containing 0.1% titanium oxide (anatase type, purity of 99.9%, manufactured by Wako Pure Chemical Industries, Ltd.), 1% silver (particle diameter of less than 100 nm, purity of 99.5%, manufactured by Sigma Corporation), 1% magnesium hydroxide (purity of 95%, manufactured by Wako Pure Chemical Industries, Ltd.), and 1% slaked dolomite used in Example 3 was prepared as described below.

1 g of the titanium oxide was added to 9 mL of pure water to prepare a 10% titanium oxide suspension. Subsequently, 1 mL of the 10% titanium oxide suspension was added to 9 mL of pure water to prepare a 1% titanium oxide suspension.

1 g of the silver was added to 9 mL of pure water to prepare a 10% silver suspension.

1 g of the magnesium hydroxide was added to 9 mL of pure water to prepare a 10% magnesium hydroxide suspension.

A 10% slaked dolomite suspension was prepared.

Subsequently, the 1% titanium oxide suspension, the 10% silver suspension, and the 10% magnesium hydroxide suspension were mixed as described below.

HPF reagent (Daiichi Pure Chemicals Co., Ltd.): 0.1 µL
0.5 M phosphoric acid buffer (pH 7.0): 20 µL
Pure water: 69.9 µL
1% titanium oxide suspension, 10% silver suspension, 10% magnesium hydroxide suspension, or 10% slaked dolomite suspension: 10 µL
Accordingly, specimens for measurement containing a 0.1% titanium oxide, 1% silver, 1% magnesium hydroxide, or 1% slaked dolomite suspension were obtained.

Two specimens each of titanium oxide and silver were prepared. While one was shaded immediately after preparation, the other was irradiated with room light (fluorescent light) and then was shaded. The irradiation time with room light was 30 minutes for the titanium oxide and 1 hour for the silver. The specimens of magnesium hydroxide and slaked dolomite were maintained shaded. One hour after the preparation of the specimens, the amounts of hydroxyl radicals generated by these specimens were measured. A fluorescent plate reader (Varioskan flash, Thermo Fisher Scientific K.K.) was used for the measurements. The result is given in Table 2.

**Table 2**

| Specimen | Light irradiation condition | Amount of generated hydroxyl radicals |
|---|---|---|
| Pure water | Irradiated with room light | 0.00 |
| 0.1% titanium oxide | Irradiated with room light | 100¹⁾ |
| | Shaded | 3.92 |
| 1% silver | Irradiated with room light²⁾ | Less than 0.1 |
| | Shaded | Less than 0.1 |
| 1% silver + 0.1% titanium oxide | Shaded | 2.21 |
| 1% magnesium hydroxide | Shaded | 28.33 |
| 1% slaked dolomite | Shaded | 13.84 |

| | | |
|---|---|---|
| 1) The amount of generated hydroxyl radicals when 0.1% titanium oxide was irradiated with room light for 30 minutes was assumed to be 100%. 2) Room light was radiated for 1 hour. | | |

These results revealed that unlike titanium oxide and silver, the method for generating hydroxyl radicals in this specification generated hydroxyl radicals even without light irradiation, that is, exerted anti-viral effects even under shaded conditions.

Example 15 Comparison of amount of generated hydroxyl radicals using metal oxides and hydroxides
Solutions as indicated in Table 3 below containing a total of 1% by weight of a metal oxide, a hydroxide, or a mixture thereof were prepared.

1 g of calcium hydroxide (purity of 96%, manufactured by Wako Pure Chemical Industries, Ltd.) was added to 9 mL of pure water to prepare a 10% calcium hydroxide suspension.

1 g of magnesium oxide (heavy, purity of 99%, manufactured by Wako Pure Chemical Industries, Ltd.) was added to 9 mL of pure water to prepare a 10% magnesium oxide suspension.

0.45g of sodium hydroxide (purity of 97%, manufactured by Wako Pure Chemical Industries, Ltd.) was added to 4.05 mL of pure water to prepare a 10% sodium hydroxide solution.

0.45g of potassium hydroxide (purity of 85%, manufactured by Sigma Corporation) was added to 4.05 mL of pure water to prepare a 10% potassium hydroxide solution.

0.5g of copper oxide (particle diameter of less than 5 µm, purity of 98%, manufactured by Sigma Corporation) was added to 4.5 mL of pure water to prepare a 10% copper oxide suspension.

For a 10% magnesium hydroxide suspension and a 10% slaked dolomite suspension, the suspensions used in Example 14 were used.

Subsequently, specimens for measurement were mixed as described below.

HPF reagent (Daiichi Pure Chemicals Co., Ltd.): 0.1 µL
0.5 M phosphoric acid buffer (pH 7.0): 20 µL
Pure water: 69.9 µL
Each suspension: 10 µL
Table 3 indicates the concentrations and amounts of a metal oxide and/or a hydroxide in each specimen to be measured.

**Table 3**

| Specimen | Content |
|---|---|
| Pure water | Pure water 10 µL |
| Slaked dolomite used in Example 3 (1%) | 10% slaked dolomite suspension 10 µL |
| Calcium hydroxide (1%) | 10% calcium hydroxide suspension 10 µL |
| Magnesium oxide (1%) | 10% magnesium oxide suspension 10 µL |
| Magnesium hydroxide (1%) | 10% magnesium hydroxide suspension 10 µL |
| Magnesium hydroxide (0.5%) | 10% magnesium hydroxide suspension 5 µL |
| Magnesium oxide (0.5%) | 10% magnesium oxide suspension 5 µL |
| Sodium hydroxide (0.5%) | 10% sodium hydroxide solution 5 µL |
| Magnesium oxide (0.5%) | 10% magnesium oxide suspension 5 µL |
| Calcium hydroxide (0.5%) | 10% calcium hydroxide suspension 5 µL |
| Magnesium oxide (0.5%) | 10% magnesium oxide suspension 5 µL |
| Potassium hydroxide (0.5%) | 10% potassium hydroxide solution 5 µL |
| Magnesium oxide (0.5%) | 10% magnesium oxide suspension 5 µL |
| Sodium hydroxide (1%) | 10% sodium hydroxide solution 10 µL |
| Potassium hydroxide (1%) | 10% potassium hydroxide solution 10 µL |
| Copper oxide (1%) | 10% copper oxide suspension 10 µL |
| Sodium hydroxide (0.5%) | 10% sodium hydroxide solution 5 µL |
| Copper oxide (0.5%) | 10% copper oxide suspension 5 µL |

The specimens were left shaded for 1 hour after the preparation, and then the amounts of generated hydroxyl radicals were measured. The amount of generated hydroxyl radicals after a 0.1% titanium oxide solution was irradiated with room light for 30 minutes was assumed to be 100% as with Example 14 and was relatively compared with the measured amounts. The result is given in Table 4.

**Table 4**

| Specimen | Amount of generated hydroxyl radicals1) (% of control) |
|---|---|
| Pure water | 0.00 |
| Slaked dolomite used in Example 3 (1%) | 12.37 |
| Calcium hydroxide (1%) | 14.27 |
| Magnesium oxide (1%) | 15.07 |
| Magnesium hydroxide (1%) | 26.17 |
| Magnesium hydroxide (0.5%) | 23.73 |
| Magnesium oxide (0.5%) | |
| Sodium hydroxide (0.5%) | 19.65 |
| Magnesium oxide (0.5%) | |
| Calcium hydroxide (0.5%) | 18.49 |
| Magnesium oxide (0.5%) | |
| Potassium hydroxide (0.5%) | 21.96 |
| Magnesium oxide (0.5%) | |
| Sodium hydroxide (1%) | 3.29 |
| Potassium hydroxide (1%) | 2.88 |
| Copper oxide (1%) | Less than 0.1 |
| Sodium hydroxide (0.5%) | 2.62 |
| Copper oxide (0.5%) | |

Example 16 Duration of hydroxyl radical generation Each reagent was mixed as described below using a suspension of a metal oxide and/or a hydroxide in a manner similar to Example 15 to prepare a specimen to be measured.

HPF reagent (Daiichi Pure Chemicals Co., Ltd.): 0.1 µL
0.5 M phosphoric acid buffer (pH 7.0): 20 µL
Pure water: 69.9 µL
Each suspension: 10 µL
Table 5 indicates the concentrations and amounts of metal oxide and/orhydroxide in each specimen to be measured.

**Table 5**

| Specimen | Content |
|---|---|
| Pure water | Pure water 10 µL |
| Slaked dolomite used in Example 3 (1%) | 10% slaked dolomite suspension 10 µL |
| Calcium hydroxide (1%) | 10% calcium hydroxide suspension 10 µL |
| MgO 1% | 10% magnesium oxide suspension 10 µL |
| Mg(OH)₂ 1% | 10% magnesium hydroxide suspension 10 µL |
| Mg(OH)₂ 0.5% + MgO 0.5% | 10% magnesium hydroxide suspension 5 µL |
| | 10% magnesium oxide suspension 5 µL |

The reagents were shaded immediately after the preparation, and the amounts of generated hydroxyl radicals were measured after 1 hour and after 12 hours. The amount of generated hydroxyl radicals after a 0.1% titanium oxide solution was irradiated with room light for 30 minutes was assumed to be 100% as with Example 4 and was relatively compared with the measured amounts. The result is given in Table 6.

**Table 6**

| Specimen | Amount of generated hydroxyl radicals (% of control) | |
|---|---|---|
| | After 1 hour | After 12 hours |
| Pure water | 0.00 | 0.00 |
| Slaked dolomite used in Example 3 (1%) | 12.37 | 60.89 |
| Calcium hydroxide (1%) | 14.27 | 47.49 |
| EgO 1% | 15.07 | 34.15 |
| Mg(OH)₂ 1% | 26.17 | 62.18 |
| Mg(OH)₂ 0.5% + MgO 0.5% | 23.73 | 74.84 |

These results revealed that the method for generating hydroxyl radicals in this specification continuously generated a sufficient amount of hydroxyl radicals that still enabled viruses to be inactivated through a period from 1 hour after the start of generation to 12 hours after the start.

Example 17 Comparison of amount of generated hydroxyl radicals using a combination of calcium hydroxide, magnesium oxide, and magnesium hydroxide
Specimens were prepared so as to have a total weight of a metal oxide and a hydroxide of 1% by weight using magnesium oxide as the metal oxide and magnesium hydroxide and/or calcium hydroxide as the hydroxide.

Specifically, each reagent was mixed as described below to prepare a specimen to be measured using a suspension of a metal oxide and/or a hydroxide in a manner similar to Example 15.

HPF reagent (Daiichi Pure Chemicals Co., Ltd.): 0.1 µL
0.5 M phosphoric acid buffer (pH 7.0): 20 µL
Pure water: 49.9 µL
Each suspension: 30 µL
Table 7 indicates the concentrations and amounts of metal oxide and/or hydroxide in each specimen to be measured.

**Table 7**

| Ca(OH)₂ | MgO | Mg(OH)₂ | Content |
|---|---|---|---|
| (%) | (%) | (%) | |
| 0 | 0 | 1 | 10% magnesium hydroxide suspension 10 µL, pure water 20 µL |
| 0 | 0.1 | 0.9 | 1% magnesium oxide suspension 10 µL, 9% magnesium hydroxide suspension 10 µL, pure water 10 µL |
| 0 | 0.2 | 0.8 | 2% magnesium oxide suspension 10 µL, 8% magnesium hydroxide suspension 10 µL, pure water 10 µL |
| 0 | 0.3 | 0.7 | 3% magnesium oxide suspension 10 µL, 7% magnesium hydroxide suspension 10 µL, pure water 10 µL |
| 0 | 0.4 | 0.6 | 4% magnesium oxide suspension 10 µL, 6% magnesium hydroxide suspension 10 µL, pure water 10 µL |
| 0 | 0.5 | 0.5 | 5% magnesium oxide suspension 10 µL, 5% magnesium hydroxide suspension 10 µL, pure water 10 µL |
| 0 | 0.6 | 0.4 | 6% magnesium oxide suspension 10 µL, 4% magnesium hydroxide suspension 10 µL, pure water 10 µL |
| 0 | 0.7 | 0.3 | 7% magnesium oxide suspension 10 µL, 3% magnesium hydroxide suspension 10 µL, pure water 10 µL |
| 0 | 0.8 | 0.2 | 8% magnesium oxide suspension 10 µL, 2% magnesium hydroxide suspension 10 µL, pure water 10 µL |
| 0 | 0.9 | 0.1 | 9% magnesium oxide suspension 10 µL, 1% magnesium hydroxide suspension 10 µL, pure water 10 µL |
| 0 | 1 | 0 | 10% magnesium hydroxide suspension 10 µL, pure water 20 µL |
| 0.1 | 0 | 0.9 | 1% Calcium hydroxide suspension 10 µL, 9% magnesium hydroxide suspension 10 µL, pure water 10 µL |
| 0.1 | 0.1 | 0.8 | 1% calcium hydroxide suspension 10 µL, 1% magnesium oxide suspension 10 µL, 8% magnesium hydroxide suspension 10 µL |
| 0.1 | 0.2 | 0.7 | 1% calcium hydroxide suspension 10 µL, 2% magnesium oxide suspension 1.0 µL, 7% magnesium hydroxide suspension 10 µL |
| 0.1 | 0.3 | 0.6 | 1% calcium hydroxide suspension 10 µL, 3% magnesium oxide suspension 10 µL, 6% magnesium hydroxide suspension 10 µL |
| 0.1 | 0.4 | 0.5 | 1% calcium hydroxide suspension 10 µL, 4% magnesium oxide suspension 10 µL, 5% magnesium hydroxide suspension 10 µL |
| 0.1 | 0.5 | 0.4 | 1% calcium hydroxide suspension 10 µL, 5% magnesium oxide suspension 10 µL, 4% magnesium hydroxide suspension 10 µL |
| 0.1 | 0.6 | 0.3 | 1% calcium hydroxide suspension 10 µL, 6% magnesium oxide suspension 10 µL, 3% magnesium hydroxide suspension 10 µL |
| 0.1 | 0.7 | 0.2 | 1% calcium hydroxide suspension 10 µL, 7% magnesium oxide suspension 10 µL, 2% magnesium hydroxide suspension 10 µL |
| 0.1 | 0.8 | 0.1 | 1% calcium hydroxide suspension 10 µL, 8% magnesium oxide suspension 10 µL, 1% magnesium hydroxide suspension 10 µL |
| 0.1 | 0.9 | 0 | 1% calcium hydroxide suspension 10 µL, 9% magnesium oxide suspension 10 µL, pure water 10 µL |
| 0.2 | 0 | 0.8 | 2% calcium hydroxide suspension 10 µL, 8% magnesium hydroxide suspension 10 µL, pure water 10 µL |
| 0.2 | 0.1 | 0.7 | 2% calcium hydroxide suspension 10 µL, 1% magnesium oxide suspension 10 µL, 7% magnesium hydroxide suspension 10 µL |
| 0.2 | 0.2 | 0.6 | 2% calcium hydroxide suspension 10 µL, 2% magnesium oxide suspension 10 µL, 6% magnesium hydroxide suspension 10 µL |
| 0.2 | 0.3 | 0.5 | 2% calcium hydroxide suspension 10 µL, 3% magnesium oxide suspension 10 pL, 5% magnesium hydroxide suspension 10 µL |
| 0.2 | 0.4 | 0.4 | 2% calcium hydroxide suspension 10 µL, 4% magnesium oxide suspension 10 µL, 4% magnesium hydroxide suspension 10 µL |
| 0.2 | 0.5 | 0.3 | 2% calcium hydroxide suspension 10 µL, 5% magnesium oxide suspension 10 µL, 3% magnesium hydroxide suspension 10 µL |
| 0.2 | 0.6 | 0.2 | 2% calcium hydroxide suspension 10 µL, 6% magnesium oxide suspension 10 µL, 2% magnesium hydroxide suspension 10 µL |
| 0.2 | 0.7 | 0.1 | 2% calcium hydroxide suspension 10 µL, 7% magnesium oxide suspension 10 µL, 1% magnesium hydroxide suspension 10 µL |
| 0.2 | 0.8 | 0 | 2% calcium hydroxide suspension 10 µL, 8% magnesium oxide suspension 10 µL, pure water 10 µL |
| 0.3 | 0 | 0.7 | 3% calcium hydroxide suspension 10 µL, 7% magnesium hydroxide suspension 10 µL, pure water 10 µL |
| 0.3 | 0.1 | 0.6 | 3% calcium hydroxide suspension 10 µL, 1% magnesium oxide suspension 10 µL, 6% magnesium hydroxide suspension 10 µL |
| 0.3 | 0.2 | 0.5 | 3% calcium hydroxide suspension 10 µL, 2% magnesium oxide suspension 10 µL, 5% magnesium hydroxide suspension 10 µL |
| 0.3 | 0.3 | 0.4 | 3% calcium hydroxide suspension 10 µL, 3% magnesium oxide suspension 10 µL, 4% magnesium hydroxide suspension 10 µL |
| 0.3 | 0.4 | 0.3 | 3% calcium hydroxide suspension 10 µL, 4% magnesium oxide suspension 10 µL, 3% magnesium hydroxide suspension 10 µL |
| 0.3 | 0.5 | 0.2 | 3% calcium hydroxide suspension 10 µL, 5% magnesium oxide suspension 10 µL, 2% magnesium hydroxide suspension 10 µL |
| 0.3 | 0.6 | 0.1 | 3% calcium hydroxide suspension 10 µL, 6% magnesium oxide suspension 10 µL, 1% magnesium hydroxide suspension 10 µL |
| 0.3 | 0.7 | 0 | 3% calcium hydroxide suspension 10 µL, 7% magnesium oxide suspension 10 µL, pure water 10 µL |
| 0.4 | 0 | 0.6 | 4% calcium hydroxide suspension 10 µL, 6% magnesium hydroxide suspension 10 µL, pure water 10 µL |
| 0.4 | 0.1 | 0.5 | 4% calcium hydroxide suspension 10 µT, 1% magnesium oxide suspension 10 µL, 5% magnesium hydroxide suspension 10 µL |
| 0.4 | 0.2 | 0.4 | 4% calcium hydroxide suspension 10 µL, 2% magnesium oxide suspension 10 µL, 4% magnesium hydroxide suspension 10 µL |
| 0.4 | 0.3 | 0.3 | 4% calcium hydroxide suspension 10 µL, 3% magnesium oxide suspension 10 µL, 3% magnesium hydroxide suspension 10 µL |
| 0.4 | 0.4 | 0.2 | 4% calcium hydroxide suspension 10 µL, 4% magnesium oxide suspension 10 µL, 2% magnesium hydroxide suspension 10 µL |
| 0.4 | 0.5 | 0.1 | 4% calcium hydroxide suspension 10 µL, 5% magnesium oxide suspension 10 µL, 1% magnesium hydroxide suspension 10 µL |
| 0.4 | 0.6 | 0 | 4% calcium hydroxide suspension 10 µL, 6% magnesium oxide suspension 10 µL, pure water 10 µL. |
| 0.5 | 0 | 0.5 | 5% calcium hydroxide suspension 10 µL, 5% magnesium hydroxide suspension 10 µL, pure water 10 µL |
| 0.5 | 0.1 | 0.4 | 5% calcium hydroxide suspension 10 µL, 1% magnesium oxide suspension 10 µL, 4% magnesium hydroxide suspension 10 µL |
| 0.5 | 0.2 | 0.3 | 5% calcium hydroxide suspension 10 µL, 2% magnesium oxide suspension 10 µL, 3% magnesium hydroxide suspension 10 µL |
| 0.5 | 0.3 | 0.2 | 5% calcium hydroxide suspension 10 µL, 3% magnesium oxide suspension 10 µL, 2% magnesium hydroxide suspension 10 µL |
| 0.5 | 0.4 | 0.1 | 5% calcium hydroxide suspension 10 µL, 4% magnesium oxide suspension 10 µL, 1% magnesium hydroxide suspension 10 µL |
| 0.5 | 0.5 | 0 | 5% calcium hydroxide suspension 10 µL, 5% magnesium oxide suspension 10 µL, pure water 10 µL |
| 0.6 | 0 | 0.4 | 6% calcium hydroxide suspension 10 µL, 4% magnesium hydroxide suspension 10 µL, pure water 10 µL |
| 0.6 | 0.1 | 0.3 | 6% calcium hydroxide suspension 10 µL, 1% magnesium oxide suspension 10 µL, 3% magnesium hydroxide suspension 10 µL |
| 0.6 | 0.2 | 0.2 | 6% calcium hydroxide suspension 10 µL, 2% magnesium oxide suspension 10 µL, 2% magnesium hydroxide suspension 10 µL |
| 0.6 | 0.3 | 0.1 | 6% calcium hydroxide suspension 10 µL, 3% magnesium oxide suspension 10 µL, 1% magnesium hydroxide suspension 10 µL |
| 0.6 | 0.4 | 0 | 6% calcium hydroxide suspension 10 µL, 4% magnesium oxide suspension 10 µL, pure water 10 µL |
| 0.7 | 0 | 0.3 | 7% calcium hydroxide suspension 10 µL, 3% magnesium hydroxide suspension 10 µL, pure water 10 µL |
| 0.7 | 0.1 | 0.2 | 7% calcium hydroxide suspension 10 µL, 1% magnesium oxide suspension 10 µL, 2% magnesium hydroxide suspension 10 µL |
| 0.7 | 0.2 | 0.1 | 7% calcium hydroxide suspension 10 µL, 2% magnesium oxide suspension 10 µL, 1% magnesium hydroxide suspension 10 µL |
| 0.7 | 0.3 | 0 | 7% calcium hydroxide suspension 10 µL, 3% magnesium oxide suspension 10 µL, pure water 10 µL |
| 0.8 | 0 | 0.2 | 8% calcium hydroxide suspension 10 µL, 2% magnesium hydroxide suspension 10 µL, pure water 10 µL |
| 0.8 | 0.1 | 0.1 | 8% calcium hydroxide suspension 10 µL, 1% magnesium oxide suspension 10 µL, 1% magnesium hydroxide suspension 10 µL |
| 0.8 | 0.2 | 0 | 8% calcium hydroxide suspension 10 µL, 2% magnesium oxide suspension 10 µL, pure water 10 µL |
| 0.9 | 0 | 0.1 | 9% calcium hydroxide suspension 10 µL, 1% magnesium hydroxide suspension 10 µL, pure water 10 µL |
| 0.9 | 0.1 | 0 | 9% calcium hydroxide suspension 10 µL, 1% magnesium oxide suspension 10 µL, pure water 10 µL |
| 1 | 0 | 0 | 10% calcium hydroxide suspension 10 µL, pure water 20 µL |

The obtained specimens were left shaded for 72 hours after which the amounts of generated hydroxyl radicals were measured. The amount of generated hydroxyl radicals when only calcium hydroxide was used was assumed to be 100%, and was relatively compared with the measured amounts. The result is given in Table 8.

**Table 8**

| Calcium hydroxide (%) | Magnesium oxide (%) | Magnesium hydroxide (%) | Amount of generated hydroxyl radicals % of activity in Ca(OH)₂ |
|---|---|---|---|
| 0 | 0 | 1 | 144.14 |
| 0 | 0.1 | 0.9 | 139.94 |
| 0 | 0.2 | 0.8 | 153.58 |
| 0 | 0.3 | 0.7 | 161.04 |
| 0 | 0.4 | 0.6 | 144.24 |
| 0 | 0.5 | 0.5 | 158.43 |
| 0 | 0.6 | 0.4 | 143.28 |
| 0 | 0.7 | 0.3 | 124.64 |
| 0 | 0.8 | 0.2 | 149.40 |
| 0 | 0.9 | 0.1 | 127.64 |
| 0 | 1 | 0 | 77.84 |
| 0.1 | 0 | 0.9 | 93.39 |
| 0.1 | 0.1 | 0.8 | 68.12 |
| 0.1 | 0.2 | 0.7 | 71.61 |
| 0.1 | 0.3 | 0.6 | 83.30 |
| 0.1 | 0.4 | 0.5 | 97.37 |
| 0.1 | 0.5 | 0.4 | 99.97 |
| 0.1 | 0.6 | 0.3 | 98.33 |
| 0.1 | 0.7 | 0.2 | 114.02 |
| 0.1 | 0.8 | 0.1 | 104.33 |
| 0.2 | 0 | 0.8 | 106.63 |
| 0.2 | 0.1 | 0.7 | 100.61 |
| 0.2 | 0.2 | 0.6 | 127.02 |
| 0.2 | 0.3 | 0.5 | 138.58 |
| 0.2 | 0.4 | 0.4 | 135.39 |
| 0.2 | 0.5 | 0.3 | 143.35 |
| 0.2 | 0.6 | 0.2 | 149.31 |
| 0.2 | 0.7 | 0.1 | 152.09 |
| 0.3 | 0 | 0.7 | 113.13 |
| 0.3 | 0.1 | 0.6 | 135.41 |
| 0.3 | 0.2 | 0.5 | 144.06 |
| 0.3 | 0.3 | 0.4 | 148.53 |
| 0.3 | 0.4 | 0.3 | 156.05 |
| 0.3 | 0.5 | 0.2 | 156.39 |
| 0.3 | 0.6 | 0.1 | 149.41 |
| 0.4 | 0 | 0.6 | 118.20 |
| 0.4 | 0.1 | 0.5 | 139.40 |
| 0.4 | 0.2 | 0.4 | 143.49 |
| 0.4 | 0.3 | 0.3 | 143.22 |
| 0.4 | 0.4 | 0.2 | 157.99 |
| 0.4 | 0.5 | 0.1 | 154.80 |
| 0.5 | 0 | 0.5 | 114.27 |
| 0.5 | 0.1 | 0.4 | 133.65 |
| 0.5 | 0.2 | 0.3 | 143.75 |
| 0.5 | 0.3 | 0.2 | 104.20 |
| 0.5 | 0.4 | 0.1 | 143.53 |
| 0.6 | 0 | 0.4 | 120.27 |
| 0.6 | 0.1 | 0.3 | 138.65 |
| 0.6 | 0.2 | 0.2 | 145.73 |
| 0.6 | 0.3 | 0.1 | 146.02 |
| 0.7 | 0 | 0.3 | 112.80 |
| 0.7 | 0.1 | 0.2 | 134.13 |
| 0.7 | 0.2 | 0.1 | 139.05 |
| 0.8 | 0 | 0.2 | 112.13 |
| 0.8 | 0.1 | 0.1 | 137.81 |
| 0.9 | 0 | 0.1 | 126.98 |
| 0.1 | 0.9 | 0 | 90.00 |
| 0.2 | 0.8 | 0 | 138.24 |
| 0.3 | 0.7 | 0 | 137.77 |
| 0.4 | 0.6 | 0 | 150.10 |
| 0.5 | 0.5 | 0 | 145.80 |
| 0.6 | 0.4 | 0 | 137.51 |
| 0.7 | 0.3 | 0 | 143.17 |
| 0.8 | 0.2 | 0 | 130.71 |
| 0.9 | 0.1 | 0 | 120.47 |
| 1 | 0 | 0 | 100.00 |
| Slaked dolomite used in Example 3 | | | 110.81 |

Example 18 Generation of hydroxyl radicals using magnesium hydroxide
Magnesium hydroxide (Mg(OH)₂) (purity of 99.90%, manufactured by Wako Pure Chemical Industries, Ltd.) was pulverized to prepare magnesium hydroxide (Mg(OH)₂) powder having a primary particle diameter in a range from 1 nm inclusive to 200 nm exclusive and magnesium hydroxide (Mg(OH)₂) having a primary particle diameter in a range from 200 nm inclusive to 400 nm exclusive.

Subsequently, a specimen (specimen No. 7) including a phosphoric acid buffer (final concentration of 0.1 M), an HPF reagent (final concentration of 5 µM), magnesium hydroxide (Mg(OH)₂) having a primary particle diameter in a range from 1 nm inclusive to 200 nm exclusive was prepared.

A specimen (specimen No. 8) including a phosphoric acid buffer (final concentration of 0.1 M), an HPF reagent (final concentration of 5 µM), magnesium hydroxide (Mg(OH)₂) having a primary particle diameter in a range from 200 nm inclusive to 400 nm exclusive was also prepared.

Subsequently, each of the specimens Nos. 7 and 8 as described above was incubated at room temperature for 15 minutes, and then the fluorescence intensity was measured by a fluorescent plate reader (ARVO MX, manufactured by PerkinElmer Co., Ltd.) to quantify hydroxyl radical production.

As a result, the amounts of generated radicals were found to be: specimen No. 7 > specimen No. 8 > specimen No. 3.

Particularly, it was revealed that when primary particles were prepared to be in a range from 1 nm inclusive to 200 nm exclusive, the produced amounts of hydroxyl radicals significantly increased.

This result revealed that magnesium hydroxide (Mg(OH)₂) prepared to be magnesium hydroxide powder having primary particles in a range from 1 nm inclusive to 200 nm exclusive significantly increased the produced amounts of hydroxyl radicals, and thus exerted anti-viral effects.

When magnesium hydroxide (Mg(OH)₂) contacts gas containing carbon dioxide (CO₂), such as air, the magnesium hydroxide partially reacted with the carbon dioxide (CO₂) to become magnesium carbonate (MgCO₃).

Mg(OH)₂+CO₂→MgCO₃+H₂O

For example, when calcium carbonate (CaCO₃) was added to specimen No. 3 indicated in Table 1, the amounts of generated hydroxyl radicals lowered to 2811.

When magnesium oxide (MgO) contacts gas containing carbon dioxide (CO₂), such as air, the magnesium oxide partially reacted with the carbon dioxide (CO₂) to become magnesium carbonate (MgCO₃).

MgO+CO₂→MgCO₃

When calcium hydroxide (Ca(OH)₂) contacts gas containing carbon dioxide (CO₂), such as air, the calcium hydroxide partially reacted with the carbon dioxide (CO₂) to become calcium carbonate (CaCO₃).

Ca(OH)₂+CO₂-+CaCO₃+H₂O

When calcium oxide (CaO) contacts gas containing carbon dioxide (CO₂), such as air, the calcium oxide partially reacted with the carbon dioxide (CO₂) to become calcium carbonate (CaCO₃).

CaO+CO₂→CaCO₃

In view of the experiments described above, metal oxide powder and magnesium hydroxide powder, created according to the invention and capable of generating hydroxyl radicals inactivating viruses, are preferably preserved in a state contacting no CO₂ (state where CO₂ is blocked) during preservation.

In other words, the anti-viral material according to the invention is preferably preserved in a state contacting no CO₂ (state where CO₂ is blocked) during preservation for generating a sufficient amount of hydroxyl radicals using the material.
Examples of the method of preserving the anti-viral material according to the invention in a state contacting no CO₂ (state where CO₂ is blocked) include a method of housing the anti-viral material according to the invention in a packaging material and then vacuumizing the inside of the packaging material to be sealed, a method of housing the anti-viral material according to the invention in a packaging material, then replacing the air in the packaging material with gas containing no CO₂, for example, inert gas such as argon (Ar) and neon (Ne), nitrogen (N₂) gas, and oxygen (O₂) gas, or a gas mixture of gas containing no such gas containing no CO₂, and then sealing the packaging material, and a method of housing the anti-viral material according to the invention in a packaging material, then filling the inside of the packaging material with gas containing no CO₂, for example, inert gas such as argon (Ar) and neon (Ne), nitrogen (N₂) gas, and oxygen (O₂) gas, or a gas mixture of gas containing no such gas containing no CO₂, and then sealing the packaging material.

When the anti-viral material according to the invention housed in a packaging material in which the anti-viral material according to the invention is preserved in such a manner is used, the packaging material is opened to expose the anti-viral material according to the invention housed in the packaging material to gas containing CO₂, such as air. At this time, the metal oxide powder or the magnesium hydroxide powder both of which are components of the anti-viral material according to the invention and that can generate hydroxyl radicals inactivating viruses are not transformed into carbonate compounds, and thus can generate a sufficient amount of hydroxyl radicals for inactivating viruses using the powder.

### [Industrial Applicability]

By the method for generating hydroxyl radicals of the invention, hydroxyl radicals can be generated safely, readily, and efficiently without employing dangerous conditions being dangerous to the human body. Moreover, various viruses can be readily and clearly inactivated by placing viruses in a hydroxyl radical atmosphere and contacting the viruses with hydroxyl radicals using the anti-viral material according to the invention produced by having such a method for generating hydroxyl radicals, which benefits industry and society directly and indirectly.

## Claims

1. A method for generating hydroxyl radicals comprising contacting powder of an oxide of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide.

2. The method according to claim 1, wherein both the powder of the metal oxide and the hydroxides are contained in slaked dolomite obtained by calcining dolomite and partially hydrating the calcined dolomite.

3. The method according to claim 2, wherein the slaked dolomite is obtained by calcining a dolomite raw material at a temperature from 700°C to 1300°C for 1 to 20 hours, cooling the calcined material to room temperature, and then contacting the cooled material with 35 to 60 parts by weight of water based on 100 parts by weight of the dolomite.

4. The method according to claim 3, wherein the calcination of the slaked dolomite is performed by heating the dolomite to a temperature from 700°C to 1000°C, at a temperature increase rate of 5 to 10°C/min and maintaining the temperature for 8 to 12 hours, while air is circulated intermittently.

5. The method according to claim 1 or 2, wherein the weight ratio of the powder of the metal oxide to the hydroxides, i.e., (powder of the metal oxide)/(hydroxides) is in the range from 0.001 to 100.

6. The method according to claim 1, wherein
the powder of the metal oxide is powder of one or more oxides selected from magnesium oxide, calcium oxide, manganese dioxide, iron (II) oxide, iron (III) oxide, copper oxide, zinc oxide, or aluminum oxide, and
the hydroxides are one or more hydroxides selected from sodium hydroxide, potassium hydroxide, magnesium hydroxide, calcium hydroxide, barium hydroxide, aluminum hydroxide, or ammonium hydroxide.

7. An anti-viral material produced by having a method for generating hydroxyl radicals by contacting powder of an oxide of one or more metals selected from the group consisting of alkali metals, alkaline earth metals, metals of groups 4 to 12 of the periodic table, or aluminum with one or more hydroxides selected from alkali metal hydroxides, alkaline earth metal hydroxides, iron hydroxide, copper hydroxide, zinc hydroxide, aluminum hydroxide, or ammonium hydroxide.
